# EUROPÄISCHE PATENTSCHRIFT

(11) **EP 1 597 240 B1**
(45) Veröffentlichungstag und Bekanntmachung des Hinweises auf die Patenterteilung: **10.09.2008**
(21) Anmeldenummer: 04711303.0
(22) Anmeldetag: 14.02.2004
(51) Int. Cl.: C07D 239/94, C07D 405/12, A61K 31/55, A61P 35/00

(54) **BICYCLISCHE HETEROCYCLEN, DIESE VERBINDUNGEN ENTHALTENDE ARZNEIMITTEL, IHRE VERWENDUNG UND VERFAHREN ZU IHRER HERSTELLUNG**
BICYCLIC HETEROCYCLES, MEDICAMENTS CONTAINING SAID COMPOUNDS, USE THEREOF, AND METHOD FOR THE PRODUCTION THEREOF
HETEROCYCLES BICYCLIQUES, MEDICAMENTS LES CONTENANT, LEUR UTILISATION ET LEURS PROCEDES DE PRODUCTION

(30) Priorität: 20.02.2003 DE 10307165
(43) Veröffentlichungstag der Anmeldung: 23.11.2005
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: HIMMELSBACH, Frank, 88441 Mittelbiberach (DE); SOLCA, Flavio, 1230 Wien (AT)
(86) Internationale Anmeldenummer: PCT/EP2004/001398
(87) Internationale Veröffentlichungsnummer: WO 2004/074263

(56) Entgegenhaltungen:
- WO-A-02/50043
- DE-A- 10 042 060
- US-A1- 2002 177 601

## Beschreibung

Gegenstand der vorliegenden Erfindung sind bicyclische Heterocyclen der allgemeinen Formel 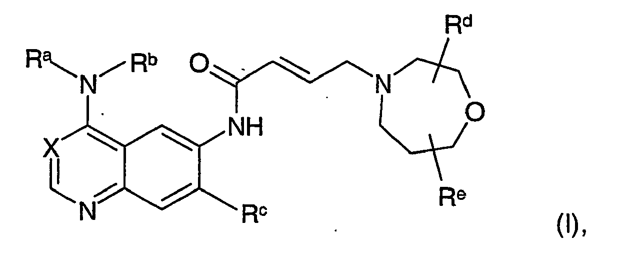 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

Chinazolinderivate zur Behandlung von Tumorerkrankungen sind in WO0250043, US2002177601 und DE 10042060 bekannt.

In der obigen allgemeinen Formel bedeutet
R^{a} ein Wasserstoffatom oder eine C₁₋₄-Alkylgruppe,
R^{b} eine Phenyl-, Benzyl- oder 1-Phenylethylgruppe, in denen der Phenylkern jeweils durch die Reste R¹ bis R³ substituiert ist, wobei
R¹ und R², die gleich oder verschieden sein können, jeweils ein Wasserstoff-, Fluor-, Chlor-, Brom- oder Jodatom,
eine C₁₋₄-Alkyl-, Hydroxy-, C₁₋₄-Alkoxy-, C₂₋₃-Alkenyl- oder C₂₋₃-Alkinylgruppe,
eine Aryl-, Aryloxy-, Arylmethyl- oder Arylmethoxygruppe,
eine Heteroaryl-, Heteroaryloxy-, Heteroarylmethyl- oder Heteroarylmethoxygruppe,
eine durch 1 bis 3 Fluoratome substituierte Methyl- oder Methoxygruppe oder
eine Cyano-, Nitro- oder Aminogruppe, und
R³ ein Wasserstoff-, Fluor-, Chlor- oder Bromatom,
eine Methyl- oder Trifluormethylgruppe darstellen,
R^{c} ein Wasserstoffatom oder ein Fluor-, Chlor- oder Bromatom,
eine Hydroxy- oder C₁₋₄-Alkyloxygruppe,
eine durch 1 bis 3 Fluoratome substituierte Ethoxygruppe,
eine durch 1 bis 5 Fluoratome substituierte Ethyloxygruppe,
eine C₂₋₄-Alkyloxygruppe, die durch einen Rest R⁴ substituiert ist, wobei
R⁴ eine Hydroxy-, C₁₋₃-Alkyloxy-, C₃₋₆Cycloalkyloxy-, C₃₋₆Cycloalkyl-C₁₋₃-alkyloxy-, Amino-, C₁₋₃-Alkylamino-, Di-(C₁₋₃-alkyl)amino-, Bis-(2-C₁₋₃-alkyloxy-ethyl)-amino-, Bis-(3-C₁₋₃-alkyloxy-propyl)-amino-, Pyrrolidin-1-yl-, Piperidin-1-yl-, Homopiperidin-1-yl-, Morpholin-4-yl-, Homomorpholin-4-yl-, Piperazin-1-yl-, 4-(C₁₋₃-Alkyl)-piporazin-1-yl-, Homopiperazin-1-yl- oder 4-(C₁₋₃-Alkyl)-homopiperazin-1-ylgruppe darstellt,
eine C₃₋₇-Cycloalkyloxy- oder C₃₋₇-Cycloalkyl-C₁₋₄-alkyloxygruppe,
eine Tetrahydrofuran-3-yloxy-, Tetrahydropyran-3-yloxy- oder Tetrahydropyran-4-yloxy-gruppe,
eine Tetrahydrofuranyl-C₁₋₄-alkyloxy- oder Tetrahydropyranyl-C₁₋₄-alkyloxygruppe,
eine Pyrrolidin-3-yloxy-, Piperidin-3-yloxy- oder Piperidin-4-yloxy-Gruppe,
eine 1-(C₁₋₃-Alkyl)-pyrrolidin-3-yloxy-, 1-(C₁₋₃-Alkyl)-piperidin-3-yloxy- oder 1-(C₁₋₃-Alkyl)-piperidin-4-yloxy-Gruppe,
eine C₁₋₄-Alkoxygruppe, die durch eine in 1-Stellung durch den Rest R⁵ substituierte Pyrrolidinyl-, Piperidinyl- oder Homopiperidinylgruppe substituiert ist, wobei
R⁵ ein Wasserstoffatom oder eine C₁₋₃-Alkylgruppe darstellt,
oder eine C₁₋₄-Alkoxygruppe, die durch eine in 4-Stellung durch den Rest R⁵ substituierte Morpholinyl- oder Homomorpholinylgruppe substituiert ist, wobei R⁵ wie vorstehend erwähnt definiert ist,
R^{e} und R^{d}, die gleich oder verschieden sein können, jeweils ein Wasserstoffatom oder eine C₁₋₃-Alkylgruppe
und
X eine durch eine Cyanogruppe substituierte Methingruppe oder ein Stickstoffatom,
wobei unter den bei der Definition der vorstehend genannten Reste erwähnten Arylgruppen jeweils eine Phenylgruppe zu verstehen ist, die durch R⁶ mono- oder disubstituiert ist, wobei die Substituenten gleich oder verschieden sein können und
R⁶ ein Wasserstoffatom, ein Fluor-, Chlor-, Brom- oder lodatom oder eine C₁₋₃-Alkyl-, Hydroxy-, C₁₋₃-Alkyloxy-, Difluormethyl-, Trifluormethyl-, Difluormethoxy-, Trifluormethoxy- oder Cyanogruppe darstellt,
unter den bei der Definition der vorstehend genannten Reste erwähnten Heteroarylgruppen eine Pyridinyl-, Pyridazinyl-, Pyrimidinyl- oder Pyrazinylgruppe zu verstehen ist, wobei die vorstehend erwähnten Heteroarylgruppen jeweils durch den Rest R⁶ mono- oder disubstituiert sind, wobei die Substituenten gleich oder verschieden sein können und R⁶ wie vorstehend erwähnt definiert ist, und
soweit nichts anderes erwähnt wurde, die vorstehend erwähnten Alkylgruppen geradkettig oder verzweigt sein können.

Bevorzugte Verbindungen der obigen allgemeinen Formel I sind diejenigen, in denen
R^{a} ein Wasserstoffatom,
R^{b} eine durch die Reste R¹ bis R³ substituierte Phenylgruppe, wobei
R¹ ein Wasserstoff-, Fluor-, Chlor- oder Bromatom,
eine Methyl-, Trifluormethyl- oder Ethinylgruppe,
eine Phenyloxy- oder Phenylmethoxygruppe, wobei der Phenylteil der vorstehend erwähnten Gruppen gegebenenfalls durch ein Fluor- oder Chloratom substituiert ist, oder
eine Pyridinyloxy- oder Pyridinylmethoxygruppe, wobei der Pyridinylteil der vorstehend erwähnten Gruppen gegebenenfalls durch eine Methyl- oder Trifluormethylgruppe substituiert ist,
R² ein Wasserstoff-, Fluor- oder Chloratom und
R³ ein Wasserstoffatom darstellen,
R^{c} ein Wasserstoffatom,
eine C₁₋₃-Alkyloxygruppe,
eine C₄₋₆-Cycloalkyloxy- oder C₃₋₆-Cycloalkyl-C₁₋₂-alkyloxy-Gruppe,
eine Tetrahydrofuran-3-yloxy-, Tetrahydropyran-3-yloxy-, Tetrahydropyran-4-yloxy-, Tetrahydrofuranyl-C₁₋₂-alkyloxy - oder Tetrahydropyranyl-C₁₋₂-alkyloxy-Gruppe,
eine Ethyloxygruppe, die in 2-Stellung durch einen Rest R⁴ substituiert ist, wobei
R⁴ eine Hydroxy-, C₁₋₃-Alkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Homopiperidin-1-yl-, Morpholin-4-yl-, Homomorpholin-4-yl-, Piperazin-1-yl-, 4-(C₁₋₃-Alkyl)-piperazin-1-yl-, Homopiperazin-1-yl-, oder 4-(C₁₋₃-Alkyl)-homopiperazin-1-yl-Gruppe darstellt,
eine Propyloxygruppe, die in 3-Stellung durch einen Rest R⁴ substituiert ist, wobei R⁴ wie vorstehend erwähnt definiert ist, oder
eine Butyloxygruppe, die in 4-Stellung durch einen Rest R⁴ substituiert ist, wobei R⁴ wie vorstehend erwähnt definiert ist,
R^{e} und R^{d}, die gleich oder verschieden sein können, jeweils ein Wasserstoffatom oder eine Methylgruppe
und
X ein Stickstoffatom bedeuten,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Besonders bevorzugte Verbindungen der obigen allgemeinen Formel I sind diejenigen, in denen
R^{a} ein Wasserstoffatom,
R^{b} eine 3-Ethinylphenyl-, 3-Bromphenyl-, 3,4-Difluorphenyl- oder 3-Chlor-4-fluor-phenylgruppe,
R^{c} ein Wasserstoffatom,
eine Methoxy-, Ethyloxy-, 2-(Methoxy)ethyloxy-, 3-(Morpholin-4-yl)propyloxy-, Cyclobutyloxy-, Cyclopentyloxy-, Cyclohexyloxy-, Cyclopropylmethoxy-, Cyclopentylmethoxy-, Tetrahydrofuran-3-yloxy-, Tetrahydropyran-3-yloxy-, Tetrahydropyran-4-yloxy-, Tetrahydrofuran-2-ylmethoxy-, Tetrahydrofuran-3-ylmethoxy- oder Tetrahydropyran-4-yl-methoxy-Gruppe,
R^{e} und R^{d} jeweils ein Wasserstoffatom
und
X ein Stickstoffatom bedeuten,
deren Tautomere, deren Stereoisomere, deren Gemische und deren Salze.

Ganz besonders bevorzugte Verbindungen der allgemeinen Formel I sind diejenigen, in denen
R^{a} ein Wasserstoffatom,
R^{b} eine 3-Chlor-4-fluor-phenylgruppe,
R^{c} eine Tetrahydrofuran-3-yloxy-Gruppe,
R^{e} und R^{d} jeweils ein Wasserstoffatom
und
X ein Stickstoffatom bedeuten,
deren Tautomere, deren Stereoisomere, deren Gemische und deren Salze.

Beispielsweise sei folgende besonders bevorzugte Verbindung der allgemeinen Formel I erwähnt:
4-[(3-Chlor-4-fluor-phenyl)amino]-6-{[4-(homomorpholin-4-yl)-1-oxo-2-buten-1-yl]amino}-7-[(*S*)-(tetrahydrofuran-3-yl)oxy]-chinazolin
sowie deren Salze.

Die Verbindungen der allgemeinen Formel I lassen sich beispielsweise nach folgenden Verfahren herstellen:
a) Umsetzung einer Verbindung der allgemeinen Formel 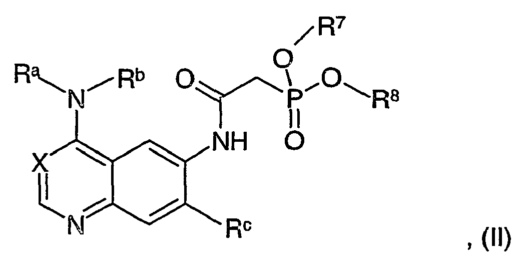 in der
   R^{a}, R^{b}, R^{c} und X wie eingangs erwähnt definiert sind und R⁷ und R⁸, die gleich oder verschieden sein können, C₁₋₄-Alkylgruppen darstellen, mit einer Verbindung der allgemeinen Formel 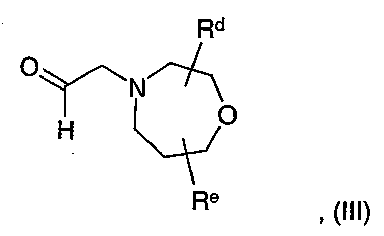 in der
   R^{d} und R^{e} wie eingangs erwähnt definiert sind.
   Die Umsetzung wird zweckmäßigerweise in einem Lösungsmittel oder Lösungsmittelgemisch wie Tetrahydrofuran, Tetrahydrofuran/Wasser, Acetonitril, Acetonitril/Wasser, Dioxan, Ethylenglycoldimethylether, Isopropanol, Methylenchlorid, Dimethylformamid oder Sulfolan gegebenenfalls in Gegenwart einer anorganischen oder organischen Base, z.B. Natriumcarbonat, Kaliumhydroxid oder 1,8-Diazabicyclo[5.4.0]undec-7-en und gegebenenfalls in Gegenwart eines Lithiumsalzes wie Lithiumchlorid bei Temperaturen zwischen -50 und 150°C, vorzugsweise jedoch bei Temperaturen zwischen -20 und 80°C, durchgeführt. Die Reaktion kann auch mit einem reaktiven Derivat der Verbindung der allgemeinen Formel III durchgeführt werden, beispielsweise dem Hydrat oder einem Hemiacetal.
b) Umsetzung einer Verbindung der allgemeinen Formel 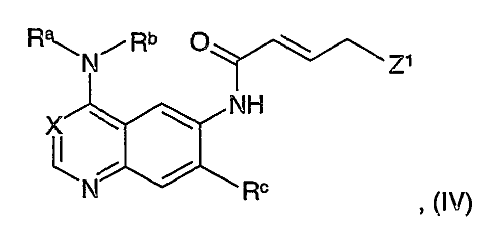 in der
   R^{a}, R^{b}, R^{c} und X wie eingangs erwähnt definiert sind und Z¹ eine Austrittsgruppe wie ein Halogenatom oder eine substituierte Sulfonyloxygruppe wie ein Chlor- oder Bromatom, eine Methansulfonyloxy- oder p-Toluolsulfonyloxygruppe darstellt, mit einer Verbindung der allgemeinen Formel 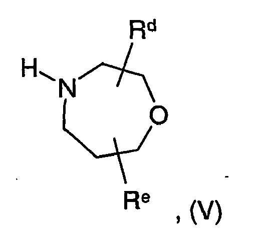 in der R^{d} und R^{e} wie eingangs erwähnt definiert ist.

Die Umsetzung wird zweckmäßigerweise in einem Lösungsmittel wie Isopropanol, Butanol, Tetrahydrofuran, Dioxan, Acetonitril, Dimethylformamid, Sulfolan, Toluol oder Methylenchlorid oder deren Gemischen gegebenenfalls in Gegenwart einer anorganischen oder organischen Base, z.B. Natriumcarbonat, Kaliumcarbonat, Kaliumhydroxid, Triethylamin oder N-Ethyl-diisopropylamin und gegebenenfalls in Gegenwart eines Reaktionsbeschleunigers wie einem Alkalijodid bei Temperaturen zwischen -20 und 150°C, vorzugsweise jedoch bei Temperaturen zwischen 0 und 100°C, durchgeführt. Die Umsetzung kann jedoch auch ohne Lösungsmittel oder in einem Überschuß der eingesetzten Verbindung der allgemeinen Formel V durchgeführt werden.

Bei den vorstehend beschriebenen Umsetzungen können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der humanen EGF-Rezeptorkinase wurde mit Hilfe der cytoplasmatischen Tyrosinkinase-Domäne (Methionin 664 bis Alanin 1186 basierend auf der in Nature 309 (1984), 418 publizierten Sequenz) bestimmt. Hierzu wurde das Protein in Sf9 Insektenzellen als GST-Fusionsprotein unter Verwendung des Baculovirus-Expressionssystems exprimiert.

Die Messung der Enzymaktivität wurde in Gegenwart oder Abwesenheit der Testverbindungen in seriellen Verdünnungen durchgeführt. Das Polymer pEY (4:1) von SIGMA wurde als Substrat verwendet. Biotinyliertes pEY (bio-pEY) wurde als Tracer-Substrat zugesetzt. Jede 100 µl Reaktionslösung enthielt 10 µl des Inhibitors in 50% DMSO, 20 µl der Substrat-Lösung (200 mM HEPES pH 7.4, 50 mM Magnesiumacetat, 2.5 mg/ml poly(EY), 5 µg/ml bio-pEY) und 20 µl Enzympräparation. Die Enzymreaktion wurde durch Zugabe von 50µl einer 100 µM ATP Lösung in 10 mM Magnesiumchlorid gestartet. Die Verdünnung der Enzympräparation wurde so eingestellt, daß der Phosphat-Einbau in das bio-pEY hinsichtlich Zeit und Enzymmenge linear war. Die Enzympräparation wurde in 20 mM HEPES pH 7.4, 1 mM EDTA, 130 mM Kochsalz, 0.05% Triton X-100, 1 mM DTT und 10% Glycerin verdünnt.

Die Enzymassays wurden bei Raumtemperatur über einen Zeitraum von 30 Minuten ausgeführt und durch Zugabe von 50 µl einer Stopplösung (250 mM EDTA in 20 mM HEPES pH 7.4) beendet. 100 µl wurden auf eine Streptavidin-beschichtete Mikrotiterplatte gebracht und 60 Minuten bei Raumtemperatur inkubiert. Danach wurde die Platte mit 200 µl einer Waschlösung (50 mM Tris, 0.05% Tween 20) gewaschen. Nach Zugabe von 100 µl eines HRPO-gelabelten anti-PY Antikörpers (PY20H Anti-PTyr:HRP von Transduction Laboratories, 250 ng/ml) wurde 60 Minuten inkubiert. Danach wurde die Mikrotiterplatte dreimal mit je 200 µl Waschlösung gewaschen. Die Proben wurden dann mit 100 µl einer TMB-Peroxidase-Lösung (A:B = 1:1, Kirkegaard Perry Laboratories) versetzt. Nach 10 Minuten wurde die Reaktion gestoppt. Die Extinktion wurde bei OD₄₅₀ₙₘ mit einem ELISA-Leser gemessen. Alle Datenpunkte wurden als Triplikate bestimmt.

Die Daten wurden mittels einer iterativen Rechnung unter Verwendung eines Analysenprogrammes (Graph Pad Prism Version 3.0; sigmoidale Kurven, variable Steigung) angepaßt. Alle Iterationsdaten wiesen einen Korrelationskoeffizienten von über 0.9 auf. Die Maxima und Minima der Kurven liegen mindestens mit einem Faktor 5 auseinander. Aus den Kurven wurde die IC₅₀. (Wirkstoffkonzentration die die Aktivität der EGF-Rezeptorkinase zu 50% hemmt) bestimmt.

Folgende Ergebnisse wurden erhalten:

| Verbindung (Beispiel Nr.) | Hemmung der EGF-Rezeptorkinase IC₅₀ [nM] |
|---|---|
| 1 | 1.5 |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome.

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch (z.B. Ambroxol, N-Acetylcystein), broncholytisch (z.B. Tiotropium oder Ipratropium oder Fenoterol, Salmeterol, Salbutamol) und/oder entzündungshemmend (z.B. Theophylline oder Glucocorticoide) wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragées, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen:

### Beispiel I

### 4-[(3-Chlor-4-fluor-phenyl)amino]-6-[(diethoxy-phosphoryl)-acetylamino]-7-[(S)-(tetrahydrofuran-3-yl)oxy]-chinazolin

60.07 g Diethoxyphosphorylessigsäure werden in 750 ml N,N-Dimethylformamid vorgelegt und bei Raumtemperatur mit 48.67 g N,N'-Carbonyldiimidazol versetzt. Nach beendeter Gasentwicklung werden 90.00 g 4-[(3-Chlor-4-fluor-phenyl)amino]-6-amino-[(*S*)-(tetrahydrofuran-3-yl)oxy]-chinazolin zugegeben und das Reaktionsgemisch wird etwa 4-5 Stunden bei Raumtemperatur gerührt, bis die Umsetzung vollständig ist. Nun wird das Reaktionsgemisch im Wasserbad leicht erwärmt und es werden zweimal je 750 ml Wasser zugegeben. Die dicke Suspension wird über Nacht nachgerührt und am nächsten Morgen werden nochmals 350 ml Wasser zugegeben. Die Suspension wird im Eisbad abgekühlt, eine Stunde nachgerührt und abgesaugt. Der Filterkuchen wird mit 240 ml N,N-Dimethylformamid/Wasser (1:2) und 240 ml Diisopropylether nachgewaschen und im Umlufttrockenschrank bei 40°C getrocknet.
Ausbeute: 117.30 g (88 % der Theorie)
R_{f}-Wert: 0.37 (Kieselgel, Methylenchlorid/Methanol = 9:1)
Massenspektrum (ESI⁺): m/z = 553, 555 [M+H]⁺

Analog Beispiel I werden folgende Verbindungen erhalten:
(1) 4-[(3-Chlor-4-fluor-phenyl)amino]-6-[(diethoxy-phosphoryl)-acetylamino]-7-[(*R*)-(tetrahydrofuran-3-yl)oxy]-chinazolin
   Massenspektrum (ESI⁺): m/z = 553, 555 [M+H]⁺
(2) 4-[(3-Chlor-4-fluor-phenyl)amino]-6-[(diethoxy-phosphoryl)-acetylamino]-7-cyclopropylmethoxy-chinazolin
   Schmelzpunkt: 185-187°C
(3) 4-[(3-Bromphenyl)amino]-6-[(diethoxy-phosphoryl)-acetylamino]-chinazolin
   Massenspektrum (ESI⁻): m/z = 491, 493 [M-H]⁻
(4) 4-[(3-Chlor-4-fluor-phenyl)amino]-6-[(diethoxy-phosphoryl)-acetylamino]-7-cyclopentyloxy-chinazolin
   R_{f}-Wert: 0.54 (Kieselgel, Methylenchlorid/Ethanol = 20:1)

### Beispiel II

### Homomorpholin-4-yl-acetaldehyd-hydrochlorid

hergestellt durch Rühren (2,5 Stunden) von 4-(2,2-Dimethoxy-ethyl)-homomorpholin mit halbkonzentrierter Salzsäure bei 80°C. Die erhaltene Lösung wird direkt unter Bsp. 1 weiter umgesetzt.

### Beispiel III

### 4-(2,2-Dimethoxy-ethyl)-homomomorpholin

hergestellt durch Rühren (5 Stunden) von Homomorpholin-hydrochlorid mit Bromacetaldehyd-dimethylacetal in Gegenwart von Kaliumcarbonat in N-Methylpyrrolidinon bei 80°C.
R_{f}-Wert: 0.2 (Kieselgel, Essigester/Methanol/konz. wässriges Ammoniak = 90:10:1)

### Herstellung der Endverbindungen:

### Beispiel 1

### 4-[(3-Chlor-4-fluor-phenyl)amino]-6-{[4-(homomorpholin-4-yl)-1-oxo-2-buten-1-yl]amino}-7-[(S)-(tetrahydrofuran-3-yl)oxy]-chinazolin

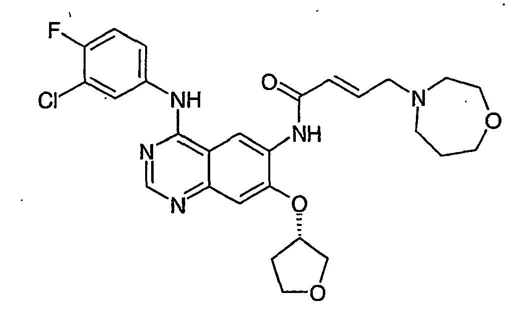

Zu einer Lösung aus 300 mg Lithiumchlorid in 20 ml Wasser wird bei Raumtemperatur eine Lösung aus 3.9 g 4-[(3-Chlor-4-fluor-phenyl)amino]-6-[2-(diethoxy-phosphoryl)-acetylamino]-7-[(S)-(tetrahydrofuran-3-yl)oxy]-chinazolin in 20 ml Tetrahydrofuran gegeben. Dann werden 2.35 g Kaliumhydroxid-Plätzchen zugesetzt und das Reaktionsgemisch wird im Eis/Aceton-Kühlbad auf -3°C abgekühlt. Nun wird die unter Beispiel II erhaltene Lösung des Homomorpholin-4-yl-acetaldehyd-hydrochlorids innehalb von 5 min bei einer Temperatur von 0°C zugetropft. Nach beendeter Zugabe wird das Reaktionsgemisch noch 10 min bei 0°C und eine weitere Stunde bei Raumtemperatur gerührt. Zur Aufarbeitung werden 100 ml Essigester zugegeben und wässrige Phase wird abgetrennt. Die organische Phase wird mit gesättigter Natriumchlorid-Lösung gewaschen, über Magnesiumsulfat getrocknet und eingeengt. Das Rohprodukt wird chromatographisch über eine Kieselgelsäule mit Essigester/Methanol/konz. methanolischem Ammoniak als Laufmittel gereinigt. Das erhaltene Produkt wird mit wenig Diisopropylether gerührt, abgesaugt und getrocknet.
Ausbeute: 2.40 g (63 % der Theorie)
R_{f}-Wert: 0.09 (Kieselgel, Essigester/Methanol/konz. wässriges Ammoniak = 90:10:1) Massenspektrum (ESI⁺): m/z = 542, 544 [M+H]⁺

Analog den vorstehend genannten Beispielen und anderen literaturbekannten Verfahren können auch folgende Verbindungen hergestellt werden:

| **Beispiel-Nr.** | **Struktur** |
|---|---|
| (1) | 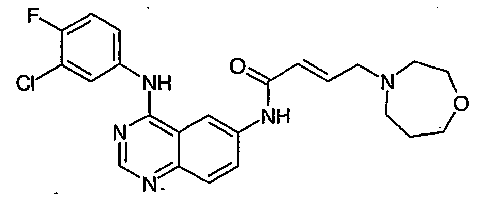 |
| (2) | 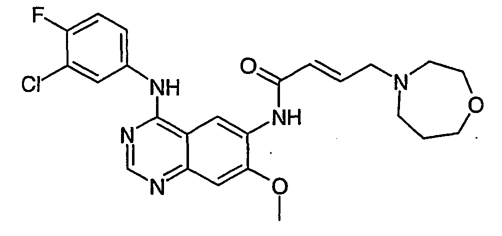 |
| (3) | 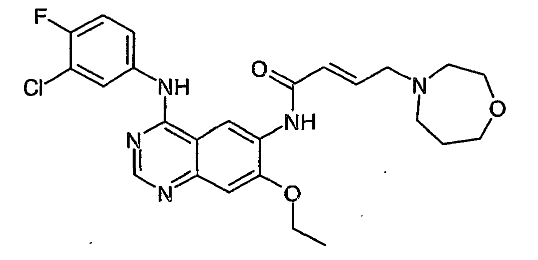 |
| (4) | 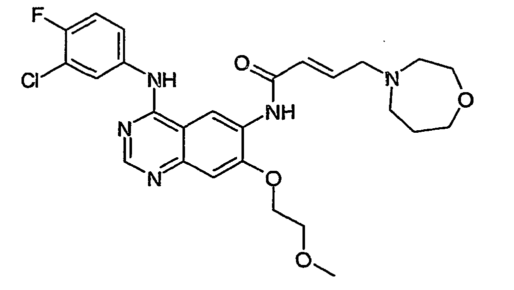 |
| (5) | 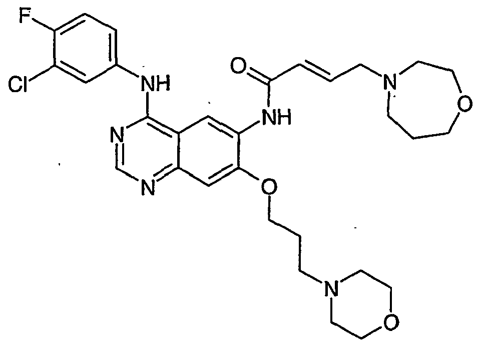 |
| (6) | 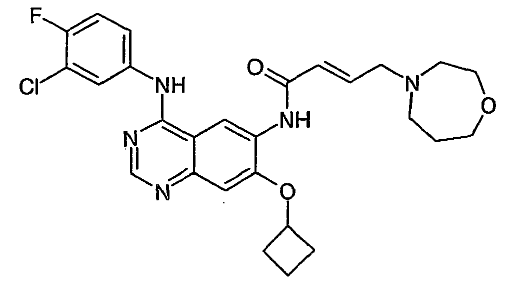 |
| (7) | 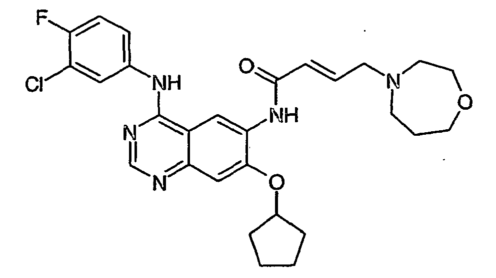 |
| (8) | 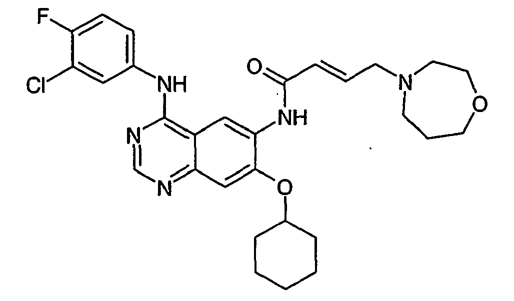 |
| (9) | 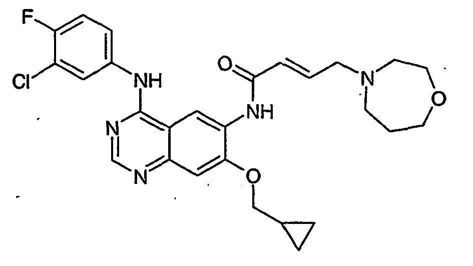 |
| (10) | 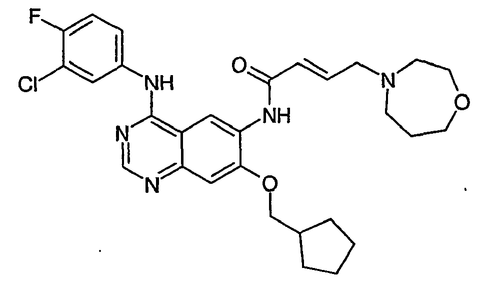 |
| (11) | 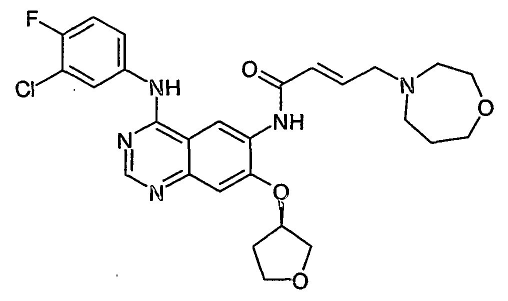 |
| (12) | 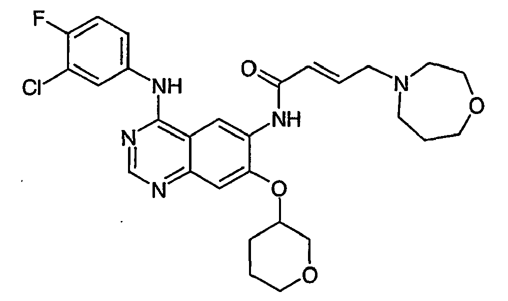 |
| (13) | 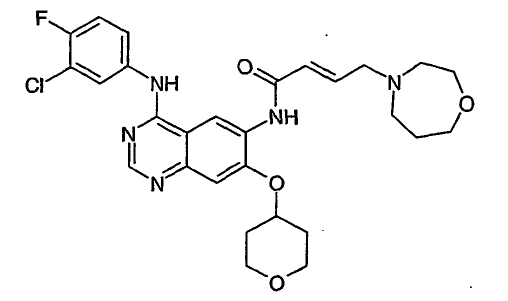 |
| (14) | 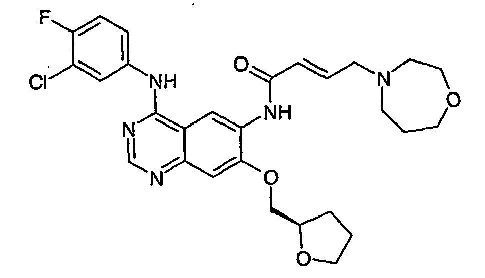 |
| (15) | 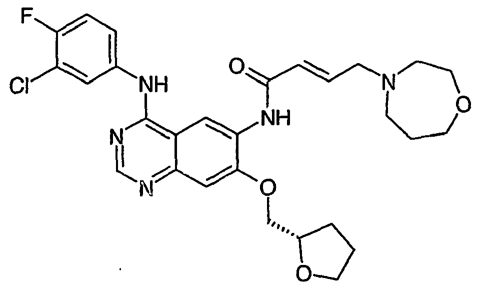 |
| (16) | 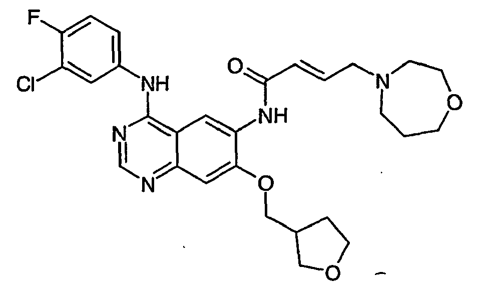 |
| (17) | 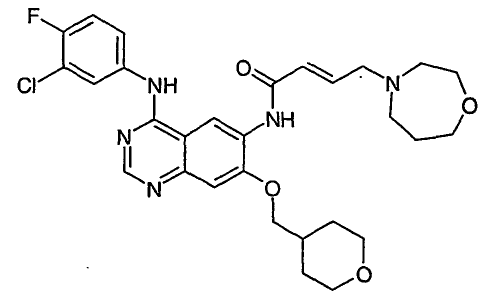 |
| (18) | 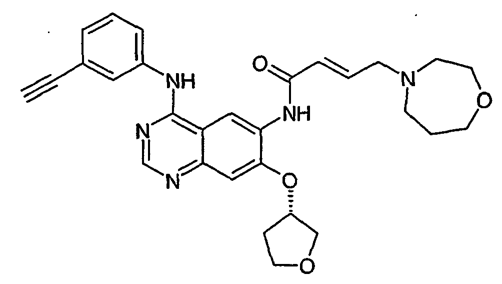 |
| (19) | 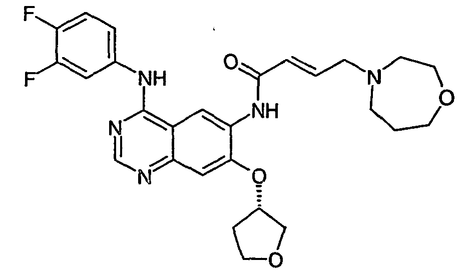 |
| (20) | 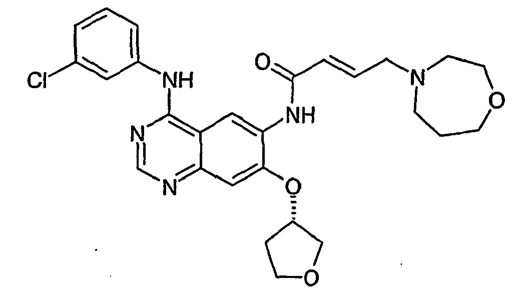 |
| (21) | 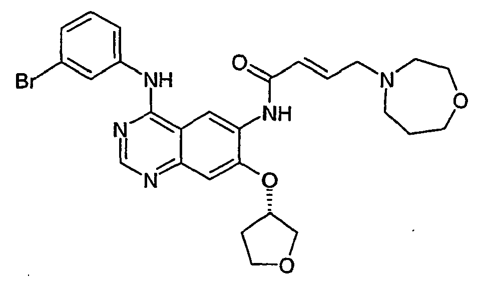 |

### Beispiel 2

| Dragées mit 75 mg Wirksubstanz | | |
|---|---|---|
| Zusammensetzung: | | |
| 1 | Dragéekern enthält: | |
| | Wirksubstanz | 75.0 mg |
| | Calciumphosphat | 93.0 mg |
| | Maisstärke | 35.5 mg |
| | Polyvinylpyrrolidon | 10.0 mg |
| | Hydroxypropylmethylcellulose | 15.0 mg |
| | Magnesiumstearat | 1.5 mg |
| | | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 3

| Tabletten mit 100 mg Wirksubstanz | | |
|---|---|---|
| Zusammensetzung: | | |
| 1 | Tablette enthält: | |
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette und einseitiger Teilkerbe. |

### Beispiel 4

| Tabletten mit 150 mg Wirksubstanz | | |
|---|---|---|
| Zusammensetzung: | | |
| 1 | Tablette enthält: | |
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | | |
|---|---|---|
| Tablettengewicht: | 300 | mg |
| Stempel: | 10 | mm, flach |

### Beispiel 5

| Hartgelatine-Kapseln mit 150 mg Wirksubstanz | | |
|---|---|---|
| Zusammensetzung: | | |
| 1 | Kapsel enthält: | |
| | Wirkstoff | 150.0 mg |
| | Maisstärke getr. ca. | 180.0 mg |
| | Milchzucker pulv. ca. | 87.0 mg |
| | Magnesiumstearat | 3.0 mg |
| | ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 6

| Suppositorien mit 150 mg Wirksubstanz | | |
|---|---|---|
| Zusammensetzung: | | |
| 1 | Zäpfchen enthält: | |
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel 7

| Suspension mit 50 mg Wirksubstanz | | |
|---|---|---|
| Zusammensetzung: | | |
| 100 ml | Suspension enthalten: | |
| | Wirkstoff | 1.00 g |
| | Carboxymethylcellulose-Na-Salz | 0.10 g |
| | p-Hydroxybenzoesäuremethylester | 0.05 g |
| | p-Hydroxybenzoesäurepropylester | 0.01 g |
| | Rohrzucker | 10.00 g |
| | Glycerin | 5.00 g |
| | Sorbitlösung 70%ig | 20.00 g |
| | Aroma | 0.30 g |
| | Wasser dest.ad 100.00 ml | |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

| Ampullen mit 10 mg Wirksubstanz | |
|---|---|
| Zusammensetzung: | |
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel 9

| Ampullen mit 50 mg Wirksubstanz | |
|---|---|
| Zusammensetzung: | |
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### Beispiel 10

| Kapseln zur Pulverinhalation mit 5 mg Wirksubstanz | |
|---|---|
| 1 Kapsel enthält: | |
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### Beispiel 11

| Inhalationslösung für Handvernebler mit 2.5 mg Wirksubstanz | |
|---|---|
| 1 Hub enthält: | |
| Wirksubstanz | 2.500 mg |
| Benzalkoniumchlorid | 0.001 mg |
| 1 N-Salzsäure q.s. | |
| Ethanol/Wasser (50/50) ad | 15.000 mg |

### Herstellung:

Die Wirksubstanz und Benzalkoniumchlorid werden in Ethanol/Wasser (50/50) gelöst. Der pH-Wert der Lösung wird mit 1 N-Salzsäure eingestellt. Die eingestellte Lösung wird filtriert und in für den Handvernebler geeignete Behälter (Kartuschen) abgefüllt.

Füllmasse des Behälters: 4.5 g

## Patentansprüche

1. Bicyclische Heterocyclen der allgemeinen Formel 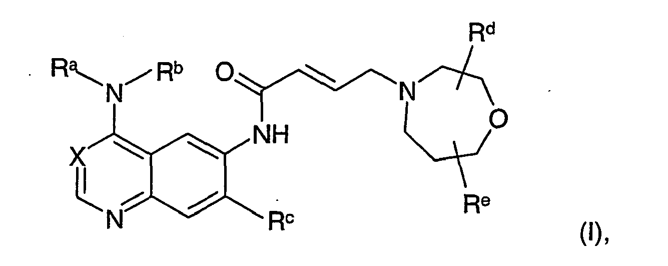 in denen
R^{a} ein Wasserstoffatom oder eine C₁₋₄-Alkylgruppe,
R^{b} eine Phenyl-, Benzyl- oder 1-Phenylethylgruppe, in denen der Phenylkern jeweils durch die Reste R¹ bis R³ substituiert ist, wobei
R¹ und R², die gleich oder verschieden sein können, jeweils ein Wasserstoff-, Fluor-, Chlor-, Brom- oder Jodatom,
eine C₁₋₄-Alkyl-, Hydroxy-, C₁₋₄-Alkoxy-, C₂₋₃-Alkenyl- oder C₂₋₃-Alkinylgruppe,
eine Aryl-, Aryloxy-, Arylmethyl- oder Arylmethoxygruppe,
eine Heteroaryl-, Heteroaryloxy-, Heteroarylmethyl- oder Heteroarylmethoxygruppe,
eine durch 1 bis 3 Fluoratome substituierte Methyl- oder Methoxygruppe oder
eine Cyano-, Nitro- oder Aminogruppe, und
R³ ein Wasserstoff-, Fluor-, Chlor- oder Bromatom,
eine Methyl- oder Trifluormethylgruppe darstellen,
R^{c} ein Wasserstoffatom oder ein Fluor-, Chlor- oder Bromatom,
eine Hydroxy- oder C₁₋₄-Alkyloxygruppe,
eine durch 1 bis 3 Fluoratome substituierte Methoxygruppe,
eine durch 1 bis 5 Fluoratome substituierte Ethyloxygruppe,
eine C₂₋₄-Alkyloxygruppe, die durch einen Rest R⁴ substituiert ist, wobei
R⁴ eine Hydroxy-, C₁₋₃-Alkyloxy-, C₃₋₆-Cycloalkyloxy-, C₃₋₆-Cycloalkyl-C₁₋₃-alkyloxy-, Amino-, C₁₋₃-Alkylamino-, Di-(C₁₋₃-alkyl)amino-, Bis-(2-C₁₋₃-alkyloxy-ethyl)-amino-, Bis-(3-C₁₋₃-alkyloxy-propyl)-amino-, Pyrrolidin-1-yl-, Piperidin-1-yl-, Homopiperidin-1-yl-, Morpholin-4-yl-, Homomorpholin-4-yl-, Piperazin-1-yl-, 4-(C₁₋₃-Alkyl)-piporazin-1-yl-, Homopiperazin-1-yl- oder 4-(C₁₋₃-Alkyl)-homopiperazin-1-ylgruppe darstellt,
eine C₃₋₇-Cycloalkyloxy- oder C₃₋₇-Cycloalkyl-C₁₋₄-alkyloxygruppe,
eine Tetrahydrofuran-3-yloxy-, Tetrahydropyran-3-yloxy- oder Tetrahydropyran-4-yloxy-gruppe,
eine Tetrahydrofuranyl-C₁₋₄-alkyloxy- oder Tetrahydropyranyl-C₁₋₄-alkyloxygruppe,
eine Pyrrolidin-3-yloxy-, Piperidin-3-yloxy- oder Piperidin-4-yloxy-Gruppe,
eine 1-(C₁₋₃-Alkyl)-pyrrolidin-3-yloxy-, 1-(C₁₋₃-Alkyl)-piperidin-3-yloxy- oder 1-(C₁₋₃-Alkyl)-piperidin-4-yloxy-Gruppe,
eine C₁₋₄-Alkoxygruppe, die durch eine in 1-Stellung durch den Rest R⁵ substituierte Pyrrolidinyl-, Piperidinyl- oder Homopiperidinylgruppe substituiert ist, wobei
R⁵ ein Wasserstoffatom oder eine C₁₋₃-Alkylgruppe darstellt,
oder eine C₁₋₄-Alkoxygruppe, die durch eine in 4-Stellung durch den Rest R⁵ substituierte Morpholinyl- oder Homomorpholinylgruppe substituiert ist, wobei R⁵ wie vorstehend erwähnt definiert ist,
R^{e} und R^{d}, die gleich oder verschieden sein können, jeweils ein Wasserstoffatom oder eine C₁₋₃-Alkylgruppe
und
X eine durch eine Cyanogruppe substituierte Methingruppe oder ein Stickstoffatom,
wobei unter den bei der Definition der vorstehend genannten Reste erwähnten Arylgruppen jeweils eine Phenylgruppe zu verstehen ist, die durch R⁶ mono- oder disubstituiert ist, wobei die Substituenten gleich oder verschieden sein können und
R⁶ ein Wasserstoffatom, ein Fluor-, Chlor-, Brom- oder lodatom oder eine C₁₋₃-Alkyl-, Hydroxy-, C₁₋₃-Alkyloxy-, Difluormethyl-, Trifluormethyl-, Difluormethoxy-, Trifluormethoxy- oder Cyanogruppe darstellt,
unter den bei der Definition der vorstehend genannten Reste erwähnten Heteroarylgruppen eine Pyridinyl-, Pyridazinyl-, Pyrimidinyl- oder Pyrazinylgruppe zu verstehen ist, wobei die vorstehend erwähnten Heteroarylgruppen jeweils durch den Rest R⁶ mono- oder disubstituiert sind, wobei die Substituenten gleich oder verschieden sein können und R⁶ wie vorstehend erwähnt definiert ist, und
soweit nichts anderes erwähnt wurde, die vorstehend erwähnten Alkylgruppen geradkettig oder verzweigt sein können,
deren Tautomere, deren Stereoisomere, deren Gemische und deren Salze.

2. Bicyclische Heterocyclen der allgemeinen Formel I gemäß Anspruch 1, in denen
R^{a} ein Wasserstoffatom,
R^{b} eine durch die Reste R¹ bis R³ substituierte Phenylgruppe, wobei
R¹ ein Wasserstoff-, Fluor-, Chlor- oder Bromatom,
eine Methyl-, Trifluormethyl- oder Ethinylgruppe,
eine Phenyloxy- oder Phenylmethoxygruppe, wobei der Phenylteil der vorstehend erwähnten Gruppen gegebenenfalls durch ein Fluor- oder Chloratom substituiert ist, oder
eine Pyridinyloxy- oder Pyridinylmethoxygruppe, wobei der Pyridinylteil der vorstehend erwähnten Gruppen gegebenenfalls durch eine Methyl- oder Trifluormethylgruppe substituiert ist,
R² ein Wasserstoff-, Fluor- oder Chloratom und
R³ ein Wasserstoffatom darstellen,
R^{c} ein Wasserstoffatom,
eine C₁₋₃-Alkyloxygruppe,
eine C₄₋₆-Cyclalkyloxy- oder C₃₋₆-Cycloalkyl-C₁₋₂-alkyloxy-Gruppe,
eine Tetrahydrofuran-3-yloxy-, Tetrahydropyran-3-yloxy-, Tetrahydropyran-4-yloxy-, Tetrahydrofuranyl-C₁₋₂-alkyloxy - oder Tetrahydropyranyl-C₁₋₂-alkyloxy-Gruppe,
eine Ethyloxygruppe, die in 2-Stellung durch einen Rest R⁴ substituiert ist, wobei
R⁴ eine Hydroxy-, C₁₋₃-Alkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Homopiperidin-1-yl-, Morpholin-4-yl-, Homomorpholin-4-yl-, Piperazin-1-yl-, 4-(C₁₋₃-Alkyl)-piperazin-1-yl-, Homopiperazin-1-yl-, oder 4-(C₁₋₃-Alkyl)-homopiperazin-1-yl-Gruppe darstellt,
eine Propyloxygruppe, die in 3-Stellung durch einen Rest R⁴ substituiert ist, wobei R⁴ wie vorstehend erwähnt definiert ist, oder
eine Butyloxygruppe, die in 4-Stellung durch einen Rest R⁴ substituiert ist, wobei R⁴ wie vorstehend erwähnt definiert ist,
R^{e} und R^{d}, die gleich oder verschieden sein können, jeweils ein Wasserstoffatom oder eine Methylgruppe
und
X ein Stickstoffatom bedeuten,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

3. Bicyclische Heterocyclen der allgemeinen Formel I gemäß Anspruch 1, in denen
R^{a} ein Wasserstoffatom,
R^{b} eine 3-Ethinylphenyl-, 3-Bromphenyl-, 3,4-Difluorphenyl- oder 3-Chlor-4-fluor-phenylgruppe,
R^{c} ein Wasserstoffatom,
eine Methoxy-, Ethyloxy-, 2-(Methoxy)ethyloxy-, 3-(Morpholin-4-yl)propyloxy-, Cyclobutyloxy-, Cyclopentyloxy-, Cyclohexyloxy-, Cyclopropylmethoxy-, Cyclopentylmethoxy-, Tetrahydrofuran-3-yloxy-, Tetrahydropyran-3-yloxy-, Tetrahydropyran-4-yloxy-, Tetrahydrofuran-2-ylmethoxy-, Tetrahydrofuran-3-ylmethoxy- oder Tetrahydropyran-4-yl-methoxy-Gruppe,
R^{e} und R^{d} jeweils ein Wasserstoffatom
und
X ein Stickstoffatom bedeuten,
deren Tautomere, deren Stereoisomere, deren Gemische und deren Salze.

4. Bicyclische Heterocyclen der allgemeinen Formel I gemäß Anspruch 1, in denen
R^{a} ein Wasserstoffatom,
R^{b} eine 3-Chlor-4-fluor-phenylgruppe,
R^{c} eine Tetrahydrofuran-3-yloxy-Gruppe,
R^{e} und R^{d} jeweils ein Wasserstoffatom
und
X ein Stickstoffatom bedeuten,
deren Tautomere, deren Stereoisomere, deren Gemische und deren Salze.

5. Folgende Verbindung der allgemeinen Formel I gemäß Anspruch 1:
4-[(3-Chlor-4-fluor-phenyl)amino]-6-{[4-(homomorpholin-4-yl)-1-oxo-2-buten-1-yl]amino}-7-[(*S*)-(tetrahydrofuran-3-yl)oxy]-chinazolin
sowie deren Salze.

6. Physiologisch verträgliche Salze der Verbindungen nach mindestens einem der Ansprüche 1 bis 5 mit anorganischen oder organischen Säuren oder Basen.

7. Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels zur Behandlung von benignen oder malignen Tumoren, zur Vorbeugung und Behandlung von Erkrankungen der Atemwege und der Lunge, zur Behandlung von Erkrankungen des Magen-Darm-Traktes oder der Gallengänge und - blase.

9. Verfahren zur Herstellung eines Arzneimittels gemäß Anspruch 7, **dadurch gekennzeichnet, daß** auf nicht-chemischem Wege eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der Verbindungen der allgemeinen Formel I gemäß den Ansprüchen 1 bis 6, **dadurch gekennzeichnet, daß**
a) eine Verbindung der allgemeinen Formel 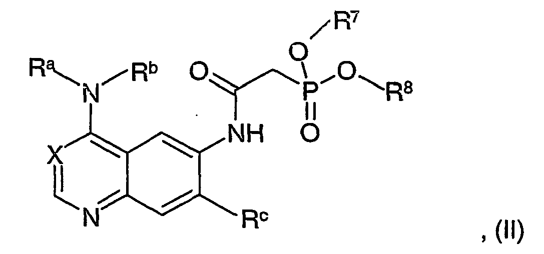 in der
R^{a}, R^{b}, R^{c} und X wie in den Ansprüchen 1 bis 5 erwähnt definiert sind und R⁷ und R⁸, die gleich oder verschieden sein können, C₁₋₄-Alkylgruppen darstellen, mit einer Verbindung der allgemeinen Formel 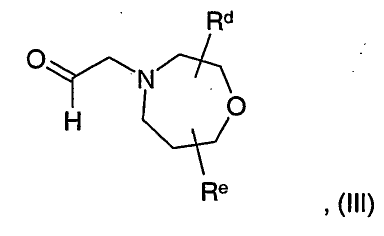 in der
R^{d} und R^{e} wie in den Ansprüchen 1 bis 5 erwähnt definiert sind, umgesetzt wird oder
b) eine Verbindung der allgemeinen Formel 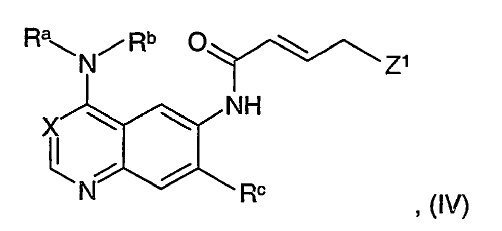 in der
R^{a}, R^{b}, R^{c} und X wie in den Ansprüchen 1 bis 5 erwähnt definiert sind und Z¹ eine Austrittsgruppe darstellt, mit einer Verbindung der allgemeinen Formel 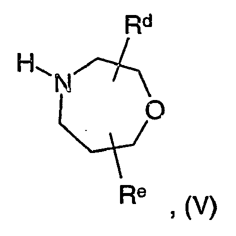 in der R^{d} und R^{e} wie in den Ansprüchen 1 bis 5 erwähnt definiert sind, umgesetzt wird und
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, übergeführt wird.

## Claims

1. Bicyclic heterocycles of general formula 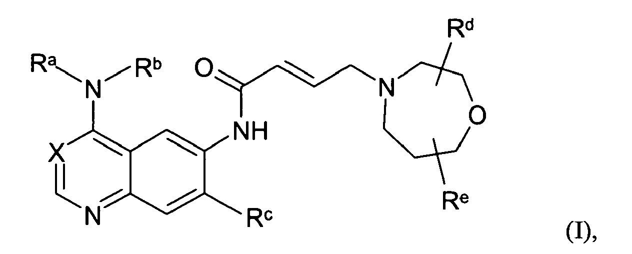 wherein
R^{a} denotes a hydrogen atom or a C₁₋₄-alkyl group,
R^{b} denotes a phenyl, benzyl or 1-phenylethyl group, wherein the phenyl nucleus in each case is substituted by the groups R¹ to R³, where
R¹ and R², which may be identical or different, in each case denote a hydrogen, fluorine, chlorine, bromine or iodine atom,
a C₁₋₄-alkyl, hydroxy, C₁₋₄-alkoxy, C₂₋₃-alkenyl or C₂₋₃-alkynyl group,
an aryl, aryloxy, arylmethyl or arylmethoxy group,
a heteroaryl, heteroaryloxy, heteroarylmethyl or heteroarylmethoxy group,
a methyl or methoxy group substituted by 1 to 3 fluorine atoms or
a cyano, nitro or amino group, and
R³ denotes a hydrogen, fluorine, chlorine or bromine atom,
a methyl or trifluoromethyl group,
R^{c} denotes a hydrogen atom or a fluorine, chlorine or bromine atom,
a hydroxy or C₁₋₄-alkyloxy group,
a methoxy group substituted by 1 to 3 fluorine atoms,
an ethyloxy group substituted by 1 to 5 fluorine atoms,
a C₂₋₄-alkyloxy group which is substituted by a group R⁴, where
R⁴ denotes a hydroxy, C₁₋₃-alkyloxy, C₃₋₆-cycloalkyloxy, C₃₋₆-cycloalkyl-C₁₋₃-alkyloxy, amino, C₁₋₃-alkylamino, di-(C₁₋₃-alkyl)amino, bis-(2-C₁₋₃-alkyloxy-ethyl)-amino, bis-(3-C₁₋₃-alkyloxy-propyl)-amino, pyrrolidin-1-yl, piperidin-1-yl, homopiperidin-1-yl, morpholin-4-yl, homomorpholin-4-yl, piperazin-1-yl, 4-(C₁₋₃-alkyl)-piperazin-1-yl, homopiperazin-1-yl or 4-(C₁₋₃-alkyl)-homopiperazin-1-yl group,
a C₃₋₇-cycloalkyloxy or C₃₋₇-cycloalkyl-C₁₋₄-alkyloxy group,
a tetrahydrofuran-3-yloxy, tetrahydropyran-3-yloxy or tetrahydropyran-4-yloxy group,
a tetrahydrofuranyl-C₁₋₄-alkyloxy or tetrahydropyranyl-C₁₋₄-alkyloxy group,
a pyrrolidin-3-yloxy, piperidin-3-yloxy or piperidin-4-yloxy group,
a 1-(C₁₋₃-alkyl)-pyrrolidin-3-yloxy, 1-(C₁₋₃-alkyl)-piperidin-3-yloxy or 1-(C₁₋₃-alkyl)-piperidin-4-yloxy group,
a C₁₋₄-alkoxy group which is substituted by a pyrrolidinyl, piperidinyl or homopiperidinyl group substituted in the 1 position by the group R⁵, where
R⁵ denotes a hydrogen atom or a C₁₋₃-alkyl group,
or a C₁₋₄-alkoxy group which is substituted by a morpholinyl or homomorpholinyl group substituted in the 4 position by the group R⁵, where R⁵ is as hereinbefore defined,
R^{e} and R^{d}, which may be identical or different, in each case denote a hydrogen atom or a C₁₋₃-alkyl group
and
X denotes a methyne group substituted by a cyano group or a nitrogen atom,
while by the aryl groups mentioned in the definition of the above groups is meant in each case a phenyl group which is mono- or disubstituted by R⁶, where the substituents may be identical or different and
R⁶ denotes a hydrogen atom, a fluorine, chlorine, bromine or iodine atom or a C₁₋₃-alkyl, hydroxy, C₁₋₃-alkyloxy, difluoromethyl, trifluoromethyl, difluoromethoxy, trifluoromethoxy or cyano group,
by the heteroaryl groups mentioned in the definition of the above groups is meant a pyridinyl, pyridazinyl, pyrimidinyl or pyrazinyl group, where the above-mentioned heteroaryl groups are mono- or disubstituted in each case by the group R⁶, where the substituents may be identical or different and R⁶ is as hereinbefore defined, and
unless otherwise stated, the above-mentioned alkyl groups may be straight-chain or branched,
the tautomers, their stereoisomers, the mixtures thereof and the salts thereof.

2. Bicyclic heterocycles of general formula I according to claim 1, wherein
R^{a} denotes a hydrogen atom,
R^{b} denotes a phenyl group substituted by the groups R¹ to R³, where
R¹ denotes a hydrogen, fluorine, chlorine or bromine atom,
a methyl, trifluoromethyl or ethynyl group,
a phenyloxy or phenylmethoxy group, where the phenyl moiety of the above-mentioned groups is optionally substituted by a fluorine or chlorine atom, or
a pyridinyloxy or pyridinylmethoxy group, where the pyridinyl moiety of the above-mentioned groups is optionally substituted by a methyl or trifluoromethyl group,
R² denotes a hydrogen, fluorine or chlorine atom and
R³ denotes a hydrogen atom,
R^{c} denotes a hydrogen atom,
a C₁₋₃-alkyloxy group,
a C₄₋₆-cycloalkyloxy or C₃₋₆-cycloalkyl-C₁₋₂-alkyloxy group,
a tetrahydrofuran-3-yloxy, tetrahydropyran-3-yloxy, tetrahydropyran-4-yloxy, tetrahydrofuranyl-C₁₋₂-alkyloxy or tetrahydropyranyl-C₁₋₂-alkyloxy group,
an ethyloxy group which is substituted in the 2 position by a group R⁴, where
R⁴ denotes a hydroxy, C₁₋₃-alkyloxy, amino, C₁₋₃-alkylamino, di-(C₁₋₃-alkyl)amino, bis-(2-methoxyethyl)-amino, pyrrolidin-1-yl, piperidin-1-yl, homopiperidin-1-yl, morpholin-4-yl, homomorpholin-4-yl, piperazin-1-yl, 4-(C₁₋₃-alkyl)-piperazin-1-yl, homopiperazin-1-yl or 4-(C₁₋₃-alkyl)-homopiperazin-1-yl group,
a propyloxy group which is substituted by a group R⁴ in the 3 position, where R⁴ is as hereinbefore defined, or
a butyloxy group which is substituted by a group R⁴ in the 4 position, where R⁴ is as hereinbefore defined,
R^{e} and R^{d}, which may be identical or different, in each case denote a hydrogen atom or a methyl group
and
X denotes a nitrogen atom,
while, unless otherwise stated, the above-mentioned alkyl groups may be straight-chain or branched,
the tautomers, their stereoisomers, the mixtures thereof and the salts thereof.

3. Bicyclic heterocycles of general formula I according to claim 1, wherein
R^{a} denotes a hydrogen atom,
R^{b} denotes a 3-ethynylphenyl, 3-bromophenyl, 3,4-difluorophenyl or 3-chloro-4-fluoro-phenyl group,
R^{c} denotes a hydrogen atom,
a methoxy, ethyloxy, 2-(methoxy)ethyloxy, 3-(morpholin-4-yl)propyloxy, cyclobutyloxy, cyclopentyloxy, cyclohexyloxy, cyclopropylmethoxy, cyclopentylmethoxy, tetrahydrofuran-3-yloxy, tetrahydropyran-3-yloxy, tetrahydropyran-4-yloxy, tetrahydrofuran-2-ylmethoxy, tetrahydrofuran-3-ylmethoxy or tetrahydropyran-4-yl-methoxy group,
R^{e} and R^{d} in each case denote a hydrogen atom
and
X denotes a nitrogen atom,
the tautomers, the stereoisomers, the mixtures thereof and the salts thereof.

4. Bicyclic heterocycles of general formula I according to claim 1, wherein
R^{a} denotes a hydrogen atom,
R^{b} denotes a 3-chloro-4-fluoro-phenyl group,
R^{c} denotes a tetrahydrofuran-3-yloxy group,
R^{e} and R^{d} in each case denote a hydrogen atom
and
X denotes a nitrogen atom,
the tautomers, their stereoisomers, the mixtures thereof and the salts thereof.

5. The following compound of general formula I according to claim 1:
4-[(3-chloro-4-fluoro-phenyl)amino]-6- {[4-(homomorpholin-4-yl)-1-oxo-2-buten-1-yl]amino}-7-[(*S*)-(tetrahydrofuran-3-yl)oxy]-quinazoline
and the salts thereof.

6. Physiologically acceptable salts of the compounds according to at least one of claims 1 to 5 with inorganic or organic acids or bases.

7. Medicaments containing a compound according to at least one of claims 1 to 5 or a physiologically acceptable salt according to claim 6, optionally together with one or more inert carriers and/or diluents.

8. Use of a compound according to at least one of claims 1 to 6 for preparing a medicament for the treatment of benign or malignant tumours, for the prevention and treatment of diseases of the airways and lungs, for the treatment of diseases of the gastro-intestinal tract or bile duct and gall bladder.

9. Process for preparing a medicament according to claim 7, **characterised in that** a compound according to at least one of claims 1 to 6 is incorporated in one or more inert carriers and/or diluents by a non-chemical method.

10. Process for preparing the compounds of general formula I according to claims 1 to 6, **characterised in that**
a) a compound of general formula 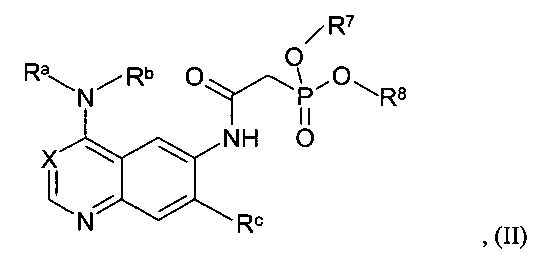 wherein
R^{a}, R^{b}, R^{c} and X are defined as in claims 1 to 5 and R⁷ and R⁸, which may be identical or different, denote C₁₋₄-alkyl groups,
is reacted with a compound of general formula 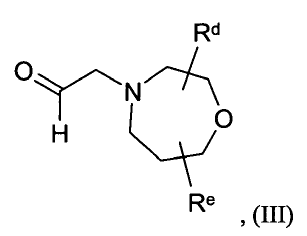 wherein
R^{d} and R^{e} are defined as in claims 1 to 5, or
b) a compound of general formula 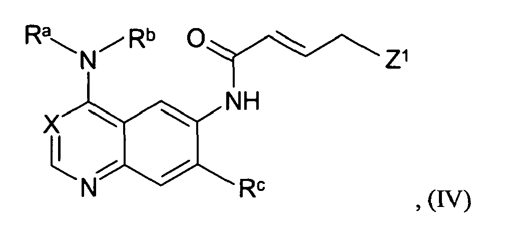
wherein
R^{a}, R^{b}, R^{c} and X are defined as in claims 1 to 5 and Z¹ denotes a leaving group, is reacted with a compound of general formula 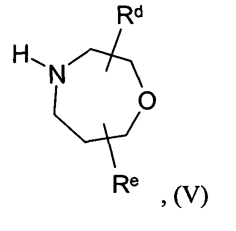 wherein R^{d} and R^{e} are defined as in claims 1 to 5, and
if necessary any protecting group used in the reactions described above is cleaved again and/or
if desired a compound of general formula I thus obtained is resolved into the stereoisomers thereof and/or
a compound of general formula I thus obtained is converted into the salts thereof, particularly for pharmaceutical use into the physiologically acceptable salts thereof.

## Revendications

1. Hétérocycles bicycliques de formule générale 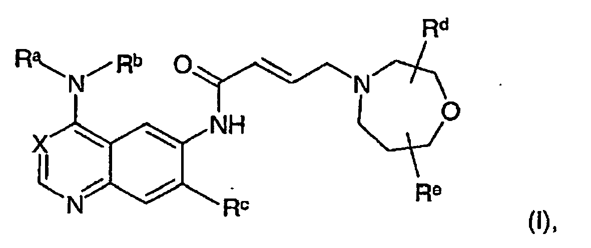 dans lesquels
R^{a} représente un atome d'hydrogène ou un groupe C₁₋₄-alkyle,
R^{b} représente un groupe phényle, benzyle ou 1-phényléthyle, dans lesquels le noyau phényle est substitué dans chaque cas par les groupements R¹ à R³, où
R¹ et R², qui peuvent être identiques ou différents, représentent dans chaque cas un atome d'hydrogène, de fluor, de chlore, de brome ou d'iode,
un groupe C₁₋₄-alkyle, hydroxyle, C₁₋₄-alcoxy, C₂₋₃-alcényle ou C₂₋₃-alcynyle,
un groupe aryle, aryloxy, arylméthyle ou arylméthoxy,
un groupe hétéroaryle, hétéroaryloxy, hétéroarylméthyle ou hétéroarylméthoxy,
un groupe méthyle ou méthoxy substitué par 1 à 3 atomes de fluor ou un groupe cyano, nitro ou amino, et
R³ représente un atome d'hydrogène, de fluor, de chlore ou de brome, un groupe méthyle ou trifluorométhyle,
R^{c} représente un atome d'hydrogène ou un atome de fluor, de chlore ou de brome,
un groupe hydroxyle ou C₁₋₄-alkyloxy,
un groupe méthoxy substitué par 1 à 3 atomes de fluor,
un groupe éthyloxy substitué par 1 à 5 atomes de fluor,
un groupe C₂₋₄-alkyloxy qui est substitué par un groupement R⁴, où
R⁴ représente un groupe hydroxyle, C₁₋₃-alkyloxy, C₃₋₆-cycloalkyloxy, C₃₋₆-cycloalkyl-C₁₋₃-alkyloxy, amino, C₁₋₃-alkylamino, di-(C₁₋₃-alkyl)amino, bis-(2-C₁₋₃-alkyloxy-éthyl)-amino, bis-(3-C₁₋₃-alkyloxy-propyl)-amino, pyrrolidin-1-yle, pipéridin-1-yle, homopipéridin-1-yle, morpholin-4-yle, homomorpholin-4-yle, pipérazin-1-yle, 4-(C₁₋₃-alkyl)-pipérazin-1-yle, homopipérazin-1-yle ou 4-(C₁₋₃-alkyl)-homopipérazin-1-yle,
un groupe C₃₋₇-cycloalkyloxy ou C₃₋₇-cycloalkyl-C₁₋₄-alkyloxy,
un groupe tétrahydrofuran-3-yloxy, tétrahydropyran-3-yloxy ou tétrahydropyran-4-yloxy,
un groupe tétrahydrofuranyl-C₁₋₄-alkyloxy ou tétrahydropyranyl-C₁₋₄-alkyloxy,
un groupe pyrrolidin-3-yloxy, pipéridin-3-yloxy ou pipéridin-4-yloxy,
un groupe 1-(C₁₋₃-alkyl)-pyrrolidin-3-yloxy, 1-(C₁₋₃-alkyl)-pipéridin-3-yloxy ou 1-(C₁₋₃-alkyl)-pipéridin-4-yloxy,
un groupe C₁₋₄-alcoxy qui est substitué par un groupe pyrrolidinyle, pipéridinyle ou homopipéridinyle substitué en position 1 par le groupement R⁵, où
R⁵ représente un atome d'hydrogène ou un groupe C₁₋₃-alkyle,
ou un groupe C₁₋₄-alcoxy qui est substitué par un groupe morpholinyle ou homomorpholinyle substitué en position 4 par le groupement R⁵, où R⁵ est défini comme indiqué précédemment,
R^{e} et R^{d}, qui peuvent être identiques ou différents, représentent chacun un atome d'hydrogène ou un groupe C₁₋₃-alkyle
et
X représente un groupe méthyne substitué par un groupe cyano, ou un atome d'azote,
où, concernant les groupes aryle cités lors de la définition des groupements cités précédemment, il convient de comprendre dans chaque cas un groupe phényle qui est mono- ou disubstitué par R⁶, où les substituants peuvent être identiques ou différents et
R⁶ représente un atome d'hydrogène, un atome de fluor, de chlore, de brome ou d'iode ou un groupe C₁₋₃-alkyle, hydroxyle, C₁₋₃-alkyloxy, difluorométhyle, trifluorométhyle, difluorométhoxy, trifluorométhoxy ou cyano,
concernant les groupes hétéroaryle cités lors de la définition des groupements cités précédemment il convient de comprendre un groupe pyridinyle, pyridazinyle, pyrimidinyle ou pyrazinyle, où les groupes hétéroaryle cités précédemment sont mono- ou disubsitués dans chaque cas par le groupement R⁶, où les substituants peuvent être identiques ou différents et R⁶ est défini comme indiqué précédemment, et
sauf indication contraire, les groupes alkyle cités précédemment peuvent être linéaires ou ramifiés,
leurs tautomères, leurs stéréoisomères, leurs mélanges et leurs sels.

2. Hétérocycles bicycliques de formule générale I selon la revendication 1, dans lesquels
R^{a} représente un atome d'hydrogène,
R^{b} représente un groupe phényle substitué par les groupements R¹ à R³, où
R¹ représente un atome d'hydrogène, de fluor, de chlore ou de brome,
un groupe méthyle, trifluorométhyle ou éthynyle,
un groupe phényloxy ou phénylméthoxy, où la partie phényle des groupes cités précédemment est éventuellement substituée par un atome de fluor ou de chlore, ou
un groupe pyridinyloxy ou pyridinylméthoxy, où la partie pyridinyle des groupes cités précédemment est éventuellement substituée par un groupe méthyle ou trifluorométhyle,
R² représente un atome d'hydrogène, de fluor ou de chlore et
R³ représente un atome d'hydrogène,
R^{c} représente un atome d'hydrogène,
un groupe C₁₋₃-alkyloxy,
un groupe C₄₋₆-cycloalkyloxy ou C₃₋₆-cycloalkyl-C₁₋₂-alkyloxy,
un groupe tétrahydrofuran-3-yloxy, tétrahydropyran-3-yloxy, tétrahydropyran-4-yloxy, tétrahydrofuranyl-C₁₋₂-alkyloxy ou tétrahydropyranyl-C₁₋₂-alkyloxy,
un groupe éthyloxy, qui est substitué en position 2 par un groupement R⁴, où
R⁴ représente un groupe hydroxyle, C₁₋₃-alkyloxy, amino, C₁₋₃-alkylamino, di-(C₁₋₃-alkyl)amino, bis-(2-méthoxyéthyl)-amino, pyrrolidin-1-yle, pipéridin-1-yle, homopipéridin-1-yle, morpholin-4-yle, homomorpholin-4-yle, pipérazin-1-yle, 4-(C₁₋₃-alkyl)-pipérazin-1-yle, homopipérazin-1-yle ou 4-(C₁₋₃-alkyl)-homopipérazin-1-yle,
un groupe propyloxy, qui est substitué en position 3 par un groupement R⁴, où R⁴ est défini comme indiqué précédemment, ou
un groupe butyloxy, qui est substitué en position 4 par un groupement R⁴, où R⁴ est défini comme indiqué précédemment,
R^{e} et R^{d}, qui peuvent être identiques ou différents, représentent chacun un atome d'hydrogène ou un groupe méthyle
et
X représente un atome d'azote,
où, sauf indication contraire, les groupes alkyle cités précédemment peuvent être linéaires ou ramifiés,
leurs tautomères, leurs stéréoisomères, leurs mélanges et leurs sels.

3. Hétérocycles bicycliques de formule générale I selon la revendication 1, dans lesquels
R^{a} représente un atome d'hydrogène,
R^{b} représente un groupe 3-éthynylphényle, 3-bromophényle, 3,4-difluorophényle
ou 3-chloro-4-fluoro-phényle,
R^{c} représente un atome d'hydrogène,
un groupe méthoxy, éthyloxy, 2-(méthoxy)éthyloxy, 3-(morpholin-4-yl)propyloxy, cyclobutyloxy, cyclopentyloxy, cyclohexyloxy, cyclopropylméthoxy, cyclopentyl-méthoxy, tétrahydrofuran-3-yloxy, tétrahydropyran-3-yloxy, tétrahydropyran-4-yloxy, tétrahydrofuran-2-ylméthoxy, tétrahydrofuran-3-ylméthoxy ou tétrahydropyran-4-ylméthoxy,
R^{e} et R^{d} représentent chacun un atome d'hydrogène
et
X représente un atome d'azote,
leurs tautomères, leurs stéréoisomères, leurs mélanges et leurs sels.

4. Hétérocycles bicycliques de formule générale I selon la revendication 1, dans lesquels
R^{a} représente un atome d'hydrogène,
R^{b} représente un groupe 3-chloro-4-fluoro-phényle,
R^{c} représente un groupe tétrahydrofuran-3-yloxy,
R^{e} et R^{d} représentent chacun un atome d'hydrogène
et
X représente un atome d'azote,
leurs tautomères, leurs stéréoisomères, leurs mélanges et leurs sels.

5. Composé de formule générale I selon la revendication 1 suivant :
4-[(3-chloro-4-fluoro-phényl)amino]-6-{[4-(homomorpholin-4-yl)-1-oxo-2-butén-1-yl]amino}-7-[(*S*)-(tétrahydrofuran-3-yl)oxy]-quinazoline
et ses sels.

6. Sels physiologiquement acceptables des composés selon au moins l'une des revendications 1 à 5 avec des acides ou bases inorganiques ou organiques.

7. Médicament contenant un composé selon au moins l'une des revendications 1 à 5 ou un sel physiologiquement acceptable selon la revendication 6 outre éventuellement un ou plusieurs vecteurs et/ou diluants inertes.

8. Utilisation d'un composé selon au moins l'une des revendications 1 à 6 pour la production d'un médicament pour le traitement des tumeurs bénignes
ou malignes, pour la prévention et le traitement des maladies des voies respiratoires et du poumon, pour le traitement des maladies des voies gastro-intestinales ou des voies biliaires et de la vésicule biliaire.

9. Procédé pour produire un médicament selon la revendication 7 **caractérisé en ce que**, par voie non chimique, un composé selon au moins l'une des revendications 1 à 6 est incorporé dans un ou plusieurs vecteurs et/ou diluants inertes.

10. Procédé pour produire les composés de formule générale I selon les revendications 1 à 6 **caractérisé en ce que**
a) un composé de formule générale 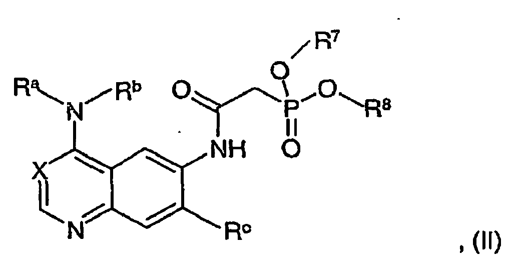 dans laquelle
R^{a}, k^{b}, R^{c} et X sont définis comme indiqué dans les revendications 1 à 5 et R⁷ et
R⁸, qui peuvent être identiques ou différents, représentent des groupes C₁₋₄-alkyle, est mis à réagir avec un composé de formule générale 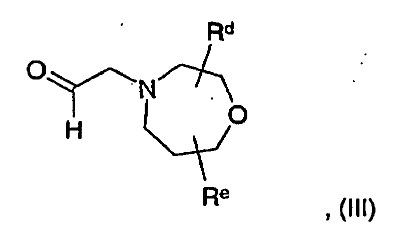 dans laquelle
R^{d} et R^{e} sont définis comme dans les revendications 1 à 5, ou
b) un composé de formule générale 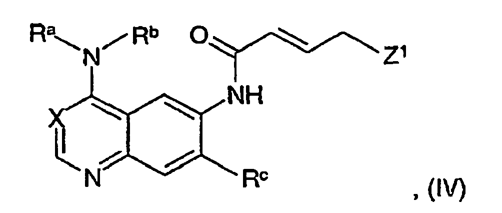
dans laquelle
R^{a}, R^{b}, R^{c} et X sont définis comme indiqué dans les revendications 1 à 5 et Z¹ représente un groupe partant, est mis à réagir avec un composé de formule générale 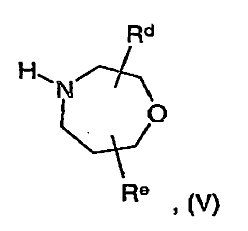 dans laquelle R^{d} et R^{e} sont définis comme dans les revendications 1 à 5,
et
si nécessaire, un groupement protecteur utilisé dans les réactions décrites précédemment est clivé et/ou
si on le souhaite, un composé de formule générale I ainsi obtenu est séparé en ses stéréoisomères et/ou
un composé de formule générale I ainsi obtenu est converti en ses sels, en particulier pour l'utilisation pharmaceutique en ses sels physiologiquement acceptables.
